# EUROPEAN PATENT SPECIFICATION

(11) **EP 1 656 915 B1**
(45) Date of publication and mention of the grant of the patent: **23.06.2010**
(21) Application number: 05110276.2
(22) Date of filing: 02.11.2005
(51) Int. Cl.: A61F 13/02

(54) **Adhesive dressing**
Haftverband
Pansement adhésif

(30) Priority: 05.11.2004 JP 2004322806
(43) Date of publication of application: 17.05.2006
(73) Proprietor: NITTO DENKO CORPORATION, Ibaraki-shi Osaka 567-8680 (JP)
(72) Inventor: Saito, Junichi c/o Nitto Denko Corporation, Osaka 567-8680/JP (JP)
(74) Representative: von Kreisler Selting Werner

(56) References cited:
- EP-A- 1 181 930
- US-A- 5 266 371

## Description

The present invention relates to an adhesive preparation superior in operability during application to the skin. More particularly, the present invention relates to an adhesive preparation that renders adhesion operation extremely easy by an improvement in the shape of a separator.

### BACKGROUND OF THE INVENTION

Adhesive preparations are used for the purpose of prophylaxis, treatment or testing of diseases. In most cases, the surface of an adhesive layer of an adhesive preparation is protected by a separator until immediately before application to the skin. A separator generally used for this purpose is obtained by applying a suitable release treatment to the surface of paper, a synthetic resin film, a metal foil or a laminate thereof. To facilitate adhesion of an adhesive preparation to the application site, moreover, a method for dividing the separator into two pieces or three pieces along parting line(s) having suitable shape(s) according to the shape, size and constitution of the adhesive preparation has been practiced or proposed.

For example, a preparation having a straight parting line dividing the separator almost in halves or in two parts having different area ratios (JP-A-59-141943), a preparation having a wavy parting line dividing the separator almost in halves (JP-A-10-265372), a preparation having approximately parallel straight or wavy parting lines dividing the separator in three parts (WO00/69422) and the like have been proposed. However, these preparations may cause difficulty in handling when in use, as described below.

To be specific, a preparation having a straight parting line dividing the separator in two parts, which is proposed in JP-A-59-141943 and used for adhesive preparations put to practice, is used as follows. First of all, one of the separator pieces divided along a parting line is peeled off from the medial end to expose an adhesive surface, the exposed adhesive surface is adhered to the skin surface, the other separator piece is peeled off from the medial end toward the outer extremity while gently sliding the piece and adhering the exposed adhesive surface to the skin surface. When the support of the preparation is flexible and lacks stiffness, the preparation is bent during application. As a result, adhesive surfaces may be adhered to each other, an adhesive surface may adhere to a non-peeled separator and the like, thus preventing proper adhesion.

In a preparation having a wavy parting line dividing the separator almost in halves as proposed in JP-A-10-265372, identifiability of the cut line in the separator is high, and a small bending causes protrusion of the wavy line. Therefore, it is superior to a preparation divided into halves along a straight parting line in the handling property. However, similar inconveniences as in the preparation described in JP-A-59-141943 may occur.

Furthermore, a preparation having straight or wavy parting lines dividing the separator in approximately parallel three parts as proposed in WO00/69422 is used as follows. First of all, a divided central separator piece is peeled off to expose the adhesive surface in the center, the exposed central adhesive surface is adhered to the skin surface, the separator pieces on the both sides are peeled off from the medial end toward the outer extremity by being gently slid while allowing the exposed adhesive surface to adhere to the skin surface. Therefore, the operability can be enhanced. However, when a non-peeled separator is held for application of the preparation to the skin after peeling off the central separator piece, more flexible support of the preparation allows easy bending of the preparation. As a result, adhesive surfaces may be adhered to each other or an adhesive surface may adhere to an non-peeled separator and the like, thus preventing proper adhesion. Therefore, the preparation described in WO00/69422 requires both hands for good adhesion and application to the shoulder, waist etc. with clothes on is extremely difficult.

### SUMMARY OF THE INVENTION

The present invention has been made in view of such situation and the problems to be solved is provision of an adhesive preparation superior in adhesion operability.

The present inventors have conducted intensive studies in an attempt to solve the aforementioned problems. As a result, they have found that an adhesive preparation comprising a separator divided into two pieces along an approximately wavy parting line and a dashed line-like cut line between the vertices of the parting line can be held or adhered with one hand, and can be extremely easily adhered to the shoulder, waist etc., which resulted in the completion of the present invention.

Accordingly, the present invention provides the following.
(1) An adhesive preparation having an about quadrate planar shape, which comprises
   a substrate layer, an adhesive layer and a separator laminated in this order, wherein
   the separator is divided into two pieces along an approximately wavy parting line, which starts from an about quadrate corner and ends at an about quadrate corner next to said corner, and the separator comprises a dashed line-like cut line between neighboring vertices of the parting line on the center side of the separator.
(2) The adhesive preparation of (1), wherein the above-mentioned cut line has a cut section having a length of 5 - 30 mm and a non-cut section having a length of 0.2 - 2 mm.
(3) The adhesive preparation of (1) or (2), wherein the shortest distance from the vertex of the above-mentioned parting line on the outer circumference side of the separator to the outer circumference of the separator is 1 - 30 mm.
(4) The adhesive preparation of any of (1) - (3), wherein the above-mentioned about quadrate is an approximate rectangle.
(5) The adhesive preparation of any of (1) - (4), wherein the above-mentioned parting line is a sine-wave.
(6) The adhesive preparation of any of (1) - (4), wherein the above-mentioned parting line is a sine-wave having an amplitude of 10 - 70 mm.
(7) The adhesive preparation of any of (1) - (6), wherein the adhesive layer contains a drug.

The adhesive preparation of the present invention is characterized in that it has an about quadrate planar shape and comprises a substrate layer, an adhesive layer and a separator laminated in this order, wherein the separator is divided into two pieces along an approximately wavy parting line, which starts from an about quadrate corner and ends at an about quadrate corner next to said corner, and the separator comprises a dashed line-like cut line between neighboring vertices of the parting line on the center side of the separator. Consequently, the preparation can be adhered with one hand without unexpected adhesion between adhesive layers or an adhesive layer and a non-peeled separator during adhering. Moreover, the separator can be extremely smoothly detached. Therefore, according to the adhesive preparation of the present invention, an adhesive preparation superior in a series of operations for adhesion can be provided.

### BRIEF DESCRIPTION OF THE DRAWINGS

Fig. 1 is a plan view showing one embodiment of the adhesive preparation of the present invention.
Fig. 2 is a sectional view along the line I-I of the adhesive preparation shown in Fig. 1.
Fig. 3 is a plan view showing the adhesive preparation of Example 1.

In the Figures, the symbols show the following items: 1 substrate layer, 3 adhesive layer, 5 separator, 7 parting line, 9 cut line, 10 adhesive preparation.

### DETAILED DESCRIPTION OF THE INVENTION

A preferable embodiment of the adhesive preparation of the present invention is explained in detail in the following. In the explanation of the Figures, the same symbol is assigned to the same element and duplicate explanation is omitted. For convenience of drawing, the size ratios of the Figures are not necessarily the same as those in the explanation.

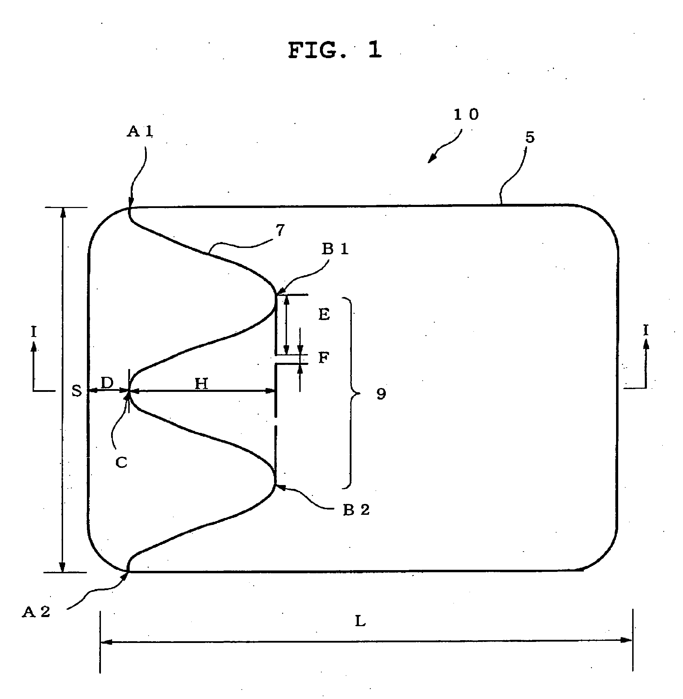

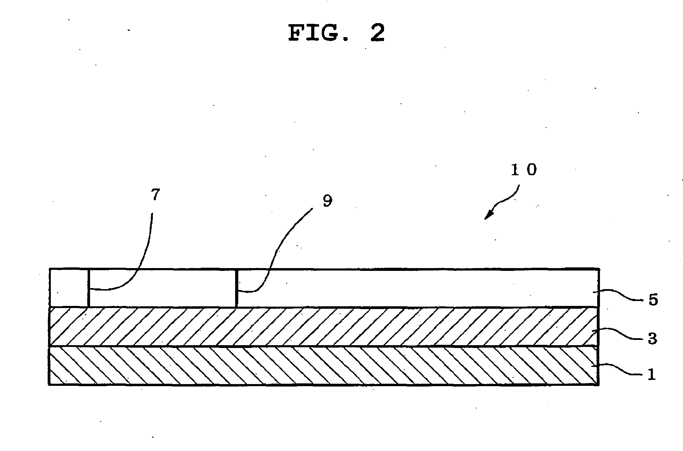

Fig. 1 is a plan view of the adhesive preparation of the present invention. Fig. 2 is a sectional view along the line I-I of the adhesive preparation shown in Fig. 1. An adhesive preparation 10 comprises a substrate layer 1, an adhesive layer 3 and a separator 5 laminated in this order. A separator 5 has a characteristic structure explained in detail in the following.

The planar shape of the adhesive preparation 10 is about quadrate. As the about quadrate, an about square, an about rectangle, a rhombus, a trapezoid and the like can be mentioned. Of these, as the planar shape of the adhesive preparation 10, an about rectangle is preferable because the effect of the present invention is particularly easily achieved. As the about rectangle, for example, a rectangle and a shape having four rounded corners can be mentioned. As used herein, the "about quadrate" is a concept encompassing a shape having four rounded corners as shown in Fig. 1. The "corner" is a concept embracing a terminal portion formed by adjacent two sides out of the 4 sides constituting an about quadrate, and, when the terminal portion is rounded to form a circular arc, the circular arc.

A shorter side S of the adhesive preparation 10 is preferably 20 mm - 150 mm, more preferably 40 mm - 120 mm. The longer side L of the adhesive preparation 10 is preferably 40 mm - 300 mm, more preferably 80 mm - 250 mm. When the shorter side S is less than 20 mm and/or the longer side L is not less than 40 mm, the adhesive preparation as a whole tends to become too small to manipulate easily with one hand and too long to handle easily. When the shorter side S exceeds 150 mm and/or the longer side L exceeds 300 mm, the preparation as a whole tends to become unnecessarily big or considerably elongated, making handling difficult.

Where necessary, the four corners of the adhesive preparation 10 may have circular arcs having a suitable curvature radius. The curvature radius is preferably 1 - 15 mm, more preferably 3 - 12 mm. When the curvature radius is less than 1 mm, the roundness causes little difference and the formation of circular arc is rather meaningless. When it exceeds 15 mm, the formation of circular arc loses significance for the preparation characteristics and tends to be economically disadvantageous because the production yield becomes low.

The separator 5 of the adhesive preparation 10 is divided into two pieces by an about wavy parting line 7. As shown in Fig. 1, the parting line 7 starts from an about quadrate corner A1 and ends at an about quadrate corner A2 next to said corner A1, which and formed on the shorter side S of the outer circumference of the adhesive preparation 10. The shape of the parting line 7 is not particularly limited as long as the adhesive surface exposed by peeling off one piece of the separator 5 can be substantially adhered to the skin. As the shape of the parting line 7, for example, trigonometric function curves such as sine-wave etc., n-th function curves such as quadratic function etc., exponential curves, logarithm function curves, periodic curves formed by semicircular arc or semiellipsoid arc, and periodic wavy lines formed by semicircular arc or semiellipsoid arc with a suitable length of straight line can be mentioned. Where necessary, these may be combined. Of these, trigonometric function curves, periodic curves formed by semicircular arc or semiellipsoid arc, and periodic wavy lines formed by semicircular arc or semiellipsoid arc with a suitable length of straight line are preferably used.

The parting line 7 has one or more vertices (e.g., C) on the outer circumference side of a separator 5 (e.g., shorter side S side) and two or more vertices (e.g., B1, B2) on the center side of the separator 5. The parting line 7 preferably has 1 - 5, more preferably 1 - 3, vertices on the shorter side S side. On the other hand, the parting line 7 preferably has 2 - 6, more preferably 2 - 4, vertices on the center side of the separator 5. When the parting line 7 has vertices in the number exceeding 6 on the center side of the separator 5, a certain amount of area of exposed adhesive layer 3 needs to be ensured so as to obtain sufficient skin adhesion on initial adhesion. For this end, a wavy shape having an extremely large amplitude is inevitable. As a result, a suitable dashed line-like cut line 9 cannot be formed easily between vertices on the center side (e.g., between B1-B2), and the operability tends to be degraded.

The amplitude H of the parting line 7 is preferably 10 - 70 mm, more preferably 20 - 60 mm. When the amplitude H is less than 10 mm, a sufficient exposed surface of adhesive layer 3 to ensure sufficient skin adhesion upon application to the skin surface cannot be obtained easily, and peeling off of the remaining non-peeled separator tends to be difficult. On the other hand, when the amplitude H exceeds 70 mm, the remaining non-peeled separator around the longer side is not delaminated clean and fine adhesion to the skin surface tends to be difficult to achieve.

To prevent the adhesive preparation 10 from bending to allow adhesion between adhesive surfaces or an adhesive surface and a non-peeled separator during operation after peeling off one piece of separator 5 of the adhesive preparation 10, the shortest distance from the vertex C of the parting line 7 on the separator outer circumference side to the outer circumference of the separator (shorter side S) needs to be sufficiently small. From this aspect, the shortest distance D from the vertex C of the parting line 7 to the shorter side S is preferably 1 - 30 mm, more preferably 2 - 20 mm. When the shortest distance D is less than 1 mm, the object of the present invention cannot be achieved sufficiently, and the production tends to be difficult. On the other hand, when the shortest distance D exceeds 30 mm, the adhesive preparation 10 bends to allow adhesion between adhesive surfaces or an adhesive surface and a non-peeled separator during adhering operation, and the desired effect of the present invention tends not to be expressed.

For application of the adhesive preparation 10, one piece of separator 5 is peeled off, the exposed adhesive surface is adhered to the application site while holding the non-peeled separator with one hand, and then the non-peeled separator is peeled off. It is preferable to form a dashed line-like cut line 9 between vertices of the about wavy parting line 7 on the center side to facilitate peeling off of the non-peeled separator. This has an effect that the non-peeled separator is easily bent suitably and can be readily held with fingers, which in turn enables to peel off the non-peeled separator easily.

The dashed line-like cut line 9 have a cut section E and a non-cut section F. The cut line 9 is preferably formed as a straight line between plural vertices (e.g., between B1-B2) on the center side of the parting line 7. The length of the cut section E is preferably 5 - 30 mm, more preferably 8 - 25 mm. The length of the non-cut section F is preferably 0.2 - 2 mm, more preferably 0.5 - 1.5 mm. The lengths of the cut section E and non-cut section F are appropriately determined depending on the size of the adhesive preparation 10 and the shape of the wavy parting line 7. When the length of the cut section E is less than 5 mm, it is difficult to make the separator suitably bend with ease unless the length of the non-cut section F is set to be considerably small and the processing also becomes complicated. On the other hand, when the length of the cut section E exceeds 30 mm, a non-uniform force applies during peeling off of the non-peeled separator and the adhesive preparation may not be applied properly or the non-cut section F may be broken. Moreover, when the length of the non-cut section F is less than 0.2 mm, the non-cut section F may be broken during peeling off of the non-peeled separator, and the adhesive preparation may not be applied properly. In addition, the processing may become very difficult. On the other hand, when the length of the non-cut section F exceeds 2 mm, the non-peeled separator may become too stiff to the extent that suitable bending with ease is difficult to achieve.

The material of the separator 5 of the adhesive preparation 10 is not particularly limited as long as it is easily peeled off from the adhesive layer 3 during use and the operability is fine during adhering. Specifically, a film of polyester, polyvinyl chloride, polyvinylidene chloride, polyethylene terephthalate etc., which is subjected to a silicone treatment of the contact surface with an adhesive layer 3, or a laminate film of polyolefin and quality paper or glassine and the like can be mentioned. Particularly, when a polyethylene terephthalate film is used, the effect of the present invention is sufficiently exerted. The thickness of the separator 5 is generally not more than 500 µm, preferably 20 - 200 µm and, from the aspect of operability during adhering, more preferably 30 - 100 µm. When the thickness of the separator 3 is less than 20 µm, stiffness becomes weak and may give rise to a problem in the operability during adhering. When it exceeds 500 µm, the separator may become too stiff or bulky, possibly giving rise to a problem in the operability during adhering.

In view of the object of the present invention, a substrate layer 1 is expected to show a higher effect when the flexibility is higher, and the material etc. thereof is not particularly limited. As the substrate layer 1, specifically, single films of polyesters (e.g., polyethylene terephthalate etc.); polyamides (e.g., nylon etc.); polyolefin (e.g., polyethylene, polypropylene etc.); polyvinyl chloride; plasticized polyvinyl chloride; plasticized vinyl acetate-vinyl chloride copolymer; polyvinylidene chloride; ethylene-vinyl acetate copolymer; cellulose acetate; ethylcellulose; ethyleneethyl acrylate copolymer; polytetrafluoroethylene; polyurethane; ionomer resin; metal foils (e.g., aluminum foil etc.); and the like or laminate films thereof and the like can be mentioned. The thickness of the substrate layer 1 made from such materials may be any as long as the softness of the adhesive preparation 10 can be maintained and it is generally 1 - 25 µm, preferably 1 - 15 µm.

To impart flexibility and stretchability, the substrate layer 1 may be made from a woven fabric, a nonwoven fabric or a knitted fabric using a synthetic resin. Particularly, the use of a knitted fabric having low bending resistance is expected to achieve a high effect of the present invention. The material of the synthetic resin is not particularly limited as long as the constituent component is impermeable and can be practically used. Specifically, viscose rayon, copper ammonia rayon, diacetate, triacetate, promix, nylon, vinylon, vinylidene, polyvinyl chloride, polyethylene terephthalate, acrylic resin, polyethylene, polypropylene, polyolefin, polyurethane, benzoate, polychlal and the like can be used. The thickness of the substrate layer 1 made from such materials is generally 100 - 2000 µm, preferably 200 - 1000 µm. Moreover, a film and a fabric may be laminated to give a substrate layer 1.

The material of the adhesive layer 3 is not particularly limited, and acrylic adhesive, natural rubber adhesive, synthetic rubber adhesive, silicone adhesive, vinyl ester adhesive, vinyl ether adhesive, acrylic or vinyl water-containing adhesive and the like conventionally used for medical applications are preferable. The thickness of the adhesive layer 3 is generally 5 - 2000 µm, preferably 10 - 1000 µm.

The adhesive layer 3 may contain a drug. Preferably, the aforementioned drug can be contained in a dissolution state, dispersion state or crystal precipitation state in the adhesive layer 3 and can transdermally express systemic or topical efficacy. As such drugs, corticosteroids, analgesic antiphlogistic, sedative hypnotic, ataractic, antihypertensive, hypotensive diuretic, antibiotic, systemic anesthesia, antibacterial agent, antifungal agent, vitamin agent, coronary vasodilator, antihistamine agent, antitussive agent, sex hormone, antidepressant, cerebral circulation activator, antiemetic agent, antitumor agent, biological drug, narcotic and the like can be specifically mentioned.

The adhesive preparation 10 can be produced by a conventionally known method. For example, a solution containing a polymer compound for forming an adhesive layer 3 is applied to a separator 5 and dried to give the adhesive layer 3, a film, a fabric and the like for forming a substrate layer 1 is adhered to one surface of the adhesive layer 3 and a separator 5 is laminated on the other surface of the adhesive layer 3 to give a whole sheet. Alternatively, a solution containing the above-mentioned polymer compound is directly applied to a film, a fabric and the like for forming a substrate layer 1 and dried to give an adhesive layer 3, and a separator 5 is laminated on the surface where the adhesive layer 3 is exposed to give a whole sheet wherein the substrate layer 1, the adhesive layer 3 and the separator 5 are laminated in this order. The adhesive preparation 10 can be produced by punching out the whole sheet obtained as mentioned above into a shape of an outer frame of the adhesive preparation, and making an about wavy parting line 7 and a dashed line-like cut line 9 in separator 5 alone with a metal blade etc.

The adhesive preparation 10 can be applied to the surfaces such as skin, mucous membrane and the like, and widely used as medical or sanitation materials, taping and the like.

### [Examples]

The present invention is explained in more detail in the following by referring to Examples, which are not to be construed as limitative.

### (Example 1)

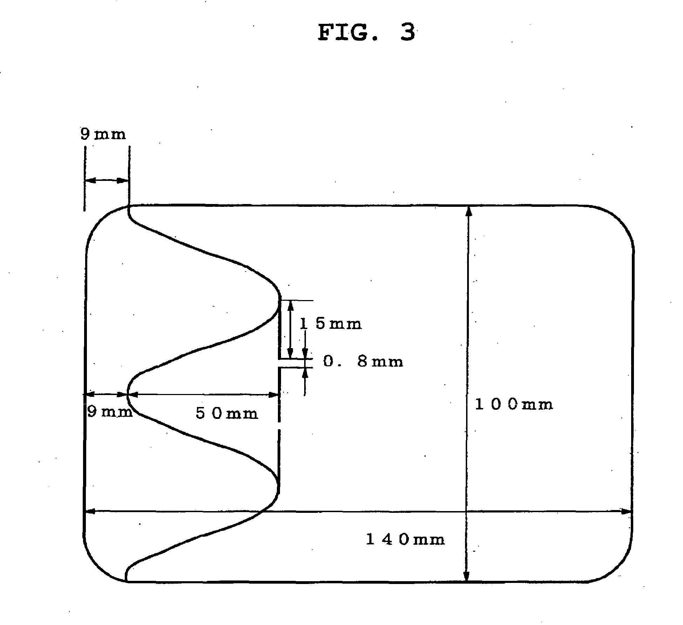

A solution of acrylic acid/octyl acrylate copolymer in ethyl acetate was applied to a 75 µm-thick polyethylene terephthalate film after a silicone release treatment, such that the thickness of the adhesive layer after drying became 80 µm, and the film was dried. Then, a polyethylene terephthalate knitted fabric (basis weight 90 g/m²) was adhered thereto to give a whole sheet. The whole sheet was punched out into a 100 mm × 140 mm about rectangle having four rounded corners with a curvature radius of 9 mm. As shown in Fig. 3, two periods of a sine-wave parting line wherein the shortest distance from the vertex on the outer circumference side of the wavy line to the shorter side is 9 mm and the amplitude is 50 mm, with the longer-side end-point of one of the two circular arcs of the shorter side as the origin of the like, and the longer-side end-point of the other circular arc of the shorter side as the end of the line, and a dashed line-like cut line comprising 15 mm long cut sections and 0.8 mm long non-cut sections between two vertices on the center side of the wavy line were formed on the separator alone with a metal blade to give an adhesive preparation.

### (Example 2)

The whole sheet obtained in Example 1 was punched out into a 100 mm×140 mm about rectangle having four rounded corners with a curvature radius of 9 mm. Then, three periods of a sine-wave parting line wherein the shortest distance from two vertices on the outer circumference side of the wavy line to the shorter side is 9 mm and the amplitude is 50 mm, with the longer-side end-point of one of the two circular arcs of the shorter side as the origin of the line, and the longer-side end-point of the other circular arc of the shorter side as the end of the line, and dashed line-like cut lines comprising 8 mm long cut sections and 0.6 mm long non-cut sections between two vertices (2 sets) on the center side of the wavy line were formed on the separator alone with a metal blade to give an adhesive preparation.

### (Example 3)

The whole sheet obtained in Example 1 was punched out into a 70 mm×100 mm about rectangle having four rounded corners with a curvature radius of 9 mm. Then, two periods of a sine-wave parting line wherein the shortest distance from the vertex on the outer circumference side of the wavy line to the shorter side is 9 mm and the amplitude is 30 mm, with the longer-side end-point of one of the two circular arcs of the shorter side as the origin of the line, and the longer-side end-point of the other circular arc of the shorter side as the end of the line, and a dashed line-like cut line comprising 8 mm long cut sections and 0.5 mm long non-cut sections between two vertices on the center side of the wavy line were formed on the separator alone with a metal blade to give an adhesive preparation.

### (Example 4)

The whole sheet obtained in Example 1 was punched out into a 70 mm×100 mm about rectangle having four rounded corners with a curvature radius of 9 mm. Then, three periods of a sine-wave parting line wherein the shortest distance from two vertices on the outer circumference side of the wavy line to the shorter side is 9 mm and the amplitude is 30 mm, with the longer-side end-point of one of the two circular arcs of the shorter side as the origin of the line, and the longer-side end-point of the other circular arc of the shorter side as the end of the line, and dashed line-like cut lines comprising 6 mm long cut sections and 0.5 mm long non-cut sections between two vertices (2 sets) on the center side of the wavy line were formed on the separator alone with a metal blade to give an adhesive preparation.

### (Example 5)

The whole sheet obtained in Example 1 was punched out into a 100 mm×140 mm about rectangle having four rounded corners with a curvature radius of 9 mm. Then, two periods of a sine-wave parting line wherein the shortest distance from the vertex on the outer circumference side to the shorter side is 35 mm and the amplitude is 50 mm, with the longer-side end-point of one of the two circular arcs of the shorter side as the origin of the line, and the longer-side end-point of the other circular arc of the shorter side as the end of the line, and a dashed line-like cut line comprising 8 mm long cut sections and 0.6 mm long non-cut sections between two vertices on the center side of the wavy line were formed on the separator alone with a metal blade to give an adhesive preparation.

### (Comparative Example 1)

The whole sheet obtained in Example 1 was punched out into a 100 mm×140 mm about rectangle having four rounded corners with a curvature radius of 9 mm. Then, three periods of a sine-wave parting line having an amplitude of 10 mm, with the center of the longer side as the origin of the line, and the center of the opposite longer side as the end of the line, was formed on the separator alone with a metal blade. In this way, an adhesive preparation having a separator divided into halves was obtained. When using this adhesive preparation, one piece of the separator was peeled off first.

### (Comparative Example 2)

The whole sheet obtained in Example 1 was punched out into a 100 mm×140 mm about rectangle having four rounded corners with a curvature radius of 9 mm. Then, two straight parting lines (20 mm intervals) were formed in the separator alone from the center of the longer side to the center of the opposite side with a metal blade. In this way, an adhesive preparation having a separator divided into three parts was obtained. When using this adhesive preparation, the central piece of the separator was first peeled off.

The adhesive preparations obtained in the above-mentioned Examples 1 - 5 and Comparative Examples 1, 2 were evaluated by 10 adults. Each of them horizontally adhered the preparation to the waist of one's own and evaluated in 4 levels with regard to the following evaluation items. An average of 10 individuals was determined for each item and the results are shown in Table 1.

### (Operability after peeling off first separator)

- 3 points: Operation with one hand is easy, without adhesion between adhesive surfaces, or an adhesive surface and a separator.
- 2 points: Operation with one hand is rather difficult, with possible adhesion adhesive surfaces, or an adhesive surface and a separator.
- 1 point: Operation with one hand is considerably difficult, with partial adhesion between adhesive surfaces, or an adhesive surface and a separator.
- 0 point: Continuation of operation with one hand is not possible, with adhesion between adhesive surfaces, or an adhesive surface and a separator.

### (Easiness of adhesion and easiness of peeling off of remaining separator)

- 3 points: A part of an adhesive surface can be easily adhered to the lesion with one hand and the remaining separator can be peeled off smoothly with one hand.
- 2 points: A part of an adhesive surface can be adhered to the lesion with one hand and the remaining separator can be peeled off with one hand.
- 1 point: Adhesion of a part of an adhesive surface to the lesion with one hand is difficult but the remaining separator can be peeled off with one hand.
- 0 point: Adhesion of a part of an adhesive surface to the lesion with one hand is difficult and the remaining separator cannot be peeled off with one hand.

### (Adhesion state)

- 3 points: Adhesion is extremely easy without wrinkles.
- 2 points: Adhesion is nearly fine though with a few wrinkles.
- 1 point: Adhesion is not fine due to a considerable number of wrinkles.
- 0 point: Adhesion is not possible.

### (Overall operability)

- 3 points: Operation is extremely easy.
- 2 points: Operation is easy.
- 1 point: Operation is rather difficult.
- 0 point: Operation is difficult.

**Table 1**

| Evaluation items | Examples | | | | | Comparative Examples | |
|---|---|---|---|---|---|---|---|
| | 1 | 2 | 3 | 4 | 5 | 1 | 2 |
| Operability after peeling off first separator | 3.0 | 2.9 | 3.0 | 3.0 | 0.8 | 0.3 | 0.5 |
| Easiness of adhesion and easiness of peeling off of remaining separator | 2.8 | 2.7 | 2.9 | 2.9 | 0.4 | 0.2 | 0.1 |
| Adhesion state | 2.8 | 2.9 | 2.7 | 2.8 | 0.9 | 0.3 | 0.2 |
| Overall operability | 2.9 | 2.8 | 2.8 | 2.9 | 0.6 | 0.2 | 0.3 |

From the above-mentioned results, the adhesive preparations of Examples 1 - 5 are superior in handling property during adhering. Particularly, unexpected adhesion between adhesive surfaces or an adhesive surface and a non-peeled separator does not occur during operation for adhering, and one-hand operation is possible. In addition, a separator can be peeled off extremely smoothly. Therefore, the adhesive preparation was confirmed to be a superior preparation, wherein a series of adhesion operations have been improved from conventional ones.

## Claims

1. An adhesive preparation having an about quadrate planar shape, which comprises
a substrate layer, an adhesive layer and a separator laminated in this order, wherein
the separator is divided into two pieces along an approximately wavy parting line, which starts from an about quadrate corner and ends at an about quadrate corner next to said corner, and
the separator comprises a dashed line-like cut line between neighboring vertices of the parting line on the center side of the separator.

2. The adhesive preparation of claim 1, wherein the above-mentioned cut line has a cut section having a length of 5 - 30 mm and a non-cut section having a length of 0.2 - 2 mm.

3. The adhesive preparation of claim 1, wherein the shortest distance from the vertex of the above-mentioned parting line on the outer circumference side of the separator to the outer circumference of the separator is 1 - 30 mm.

4. The adhesive preparation of claim 1, wherein the above-mentioned about quadrate is an approximate rectangle.

5. The adhesive preparation of claim 1, wherein the above-mentioned parting line is a sine-wave.

6. The adhesive preparation of claim 1, wherein the above-mentioned parting line is a sine-wave having an amplitude of 10 - 70 mm.

7. The adhesive preparation of claim 1, wherein the adhesive layer contains a drug.

## Patentansprüche

1. Kleberpräparat mit einer ungefähr quadratischen planaren Form, umfassend:
eine Substratschicht, eine Kleberschicht und einen Separator, die in dieser Reihenfolge miteinander laminiert sind, wobei
der Separator entlang einer ungefähr wellenförmigen Trennlinie, die an einer Ecke der ungefähr quadratischen Form beginnt und an einer dieser Ecke benachbarten Ecke der ungefähr quadratischen Form endet, in zwei Stücke unterteilt ist und
der Separator eine Schnittlinie in der Art einer gestrichelten Linie zwischen benachbarten Spitzen der Trennlinie auf der Zentrumsseite des Separators umfasst.

2. Kleberpräparat gemäß Anspruch 1, wobei die oben genannte Schnittlinie einen geschnittenen Abschnitt mit einer Länge von 5 bis 30 mm und einen nichtgeschnittenen Abschnitt mit einer Länge von 0,2 bis 2 mm aufweist.

3. Kleberpräparat gemäß Anspruch 1, wobei der kürzeste Abstand von der Spitze der oben genannten Trennlinie auf der äußeren Umfangseite des Separators zum äußeren Umfang des Separators 1 bis 30 mm beträgt.

4. Kleberpräparat gemäß Anspruch 1, wobei das oben genannte ungefähre Quadrat ein ungefähres Rechteck ist.

5. Kleberpräparat gemäß Anspruch 1, wobei die oben genannte Trennlinie eine Sinuswelle ist.

6. Kleberpräparat gemäß Anspruch 1, wobei die oben genannte Trennlinie eine Sinuswelle mit einer Amplitude von 10 bis 70 mm ist.

7. Kleberpräparat gemäß Anspruch 1, wobei die Kleberschicht einen Wirkstoff enthält.

## Revendications

1. Préparation adhésive ayant une forme plane quasi carrée, qui comprend
une couche de substrat, une couche adhésive et une couche de séparation stratifiées dans cet ordre, dans laquelle
la couche de séparation est divisée en deux le long d'une ligne de partition quasiment ondulée, qui va d'un angle quasiment carré à un autre angle quasiment carré proche dudit premier angle, et
la couche de séparation comprend une ligne de découpe en pointillés entre les sommets voisins de la ligne de partition au centre de la couche de séparation.

2. Préparation adhésive selon la revendication 1, dans laquelle la ligne de découpe susmentionnée possède une section de découpe ayant une longueur de 5 à 30 mm et une section de non-découpe ayant une longueur de 0,2 à 2 mm.

3. Préparation adhésive selon la revendication 1, dans laquelle la distance la plus courte entre le sommet de la ligne de partition susmentionnée sur le circonférence extérieure de la couche de séparation et la circonférence extérieure de la couche de séparation est de 1 à 30 mm.

4. Préparation adhésive selon la revendication 1, dans laquelle le quasi-carré susmentionné est un quasi-rectangle.

5. Préparation adhésive selon la revendication 1, dans laquelle la ligne de partition susmentionnée est une onde sinusoïdale.

6. Préparation adhésive selon la revendication 1, dans laquelle la ligne de partition susmentionnée est une onde sinusoïdale ayant une amplitude de 10 à 70 mm.

7. Préparation adhésive selon la revendication 1, dans laquelle la couche adhésive contient un médicament.
